# EUROPÄISCHE PATENTANMELDUNG

(11) **EP 4 785 886 A1**
(43) Veröffentlichungstag der Anmeldung: **05.08.2026**
(21) Anmeldenummer: 26151723.9
(22) Anmeldetag: 14.01.2026
(51) Int. Cl.: A61B 17/80, A61L 31/02

(54) **OSTEOSYNTHESEPLATTE SOWIE VERFAHREN ZUR HERSTELLUNG EINER OSTEOSYNTHESEPLATTE**

(30) Priorität: 30.01.2025 DE 102025103444
(71) Anmelder: Karl Leibinger Asset Management GmbH & Co. KG, 78570 Mühlheim an der Donau (DE)
(72) Erfinder: Pabst, Martin, 78479 Reichenau (DE); Zschocke, Iris, 77716 Haslach im Kinzigtal (DE); Merkt, Harald, 78176 Blumberg Baden (DE); Pfeiffer, Vincent, 78570 Mühlheim an der Donau (DE); Kohler, Klaus, 72505 Hausen (DE); Meer, Martin, 72189 Vöhringen (DE)
(74) Vertreter: Schwamberger, Martin

(57) **Zusammenfassung**

Osteosyntheseplatte (P) zur Fixierung von Knochenfragmenten, wobei die Osteosyntheseplatte (P) einen Grundkörper (G) mit einer Oberseite (G1) und mit einer Unterseite (G2) aufweist, wobei die Unterseite (G2) zur Auflage der Osteosyntheseplatte (P) auf den Knochenfragmenten vorgesehen ist, wobei die Osteosyntheseplatte (P) mehrere zwischen der Oberseite (G1) und der Unterseite (G2) ausgebildete Öffnungen (A) zur Aufnahme von je einer Schraube (SK) aufweist, wobei der Grundkörper (G) einen mehrschichtigen Aufbau aufweist, umfassend eine Kernschicht (K), eine erste Deckschicht (S1), welche die Kernschicht (K) auf einer ersten Seite zumindest teilweise bedeckt, und eine zweite Deckschicht (S2), welche die Kernschicht (K) auf einer der ersten Seite gegenüberliegenden zweiten Seite zumindest teilweise bedeckt, wobei die Kernschicht (K) aus einem ersten Werkstoff gefertigt ist, wobei die erste und zweite Deckschicht (S1, S2) aus einem zweiten Werkstoff gefertigt sind, wobei sich der erste Werkstoff vom zweiten Werkstoff unterscheidet, und wobei der erste Werkstoff eine höhere Duktilität aufweist als der zweite Werkstoff, sowie Verfahren zur Herstellung einer solchen Osteosyntheseplatte (P).

## Beschreibung

Die Erfindung betrifft eine Osteosyntheseplatte zur Fixierung von Knochenfragmenten, sowie ein Verfahren zur Herstellung einer solchen Osteosyntheseplatten.

Bei der Osteosynthese werden nach einer Knochenfraktur im Rahmen eines operativen Eingriffs zwei oder mehr Knochenfragmente miteinander verbunden, um ein Zusammenwachsen der Knochenfragmente zu erreichen. Ziel der Osteosynthese ist dabei eine stabile Fixierung der Knochenfragmente, wobei diese Fixierung dabei in einer korrekten Stellung der Knochenfragmente zueinander und somit ggf. unter Korrektur von Fehlstellungen erfolgen soll. Neben einer Fixierung über Draht oder Schrauben kommen je nach Anwendungsbereich auch Osteosyntheseplatten zur Anwendung, wobei die jeweilige Osteosyntheseplatte dabei im Bereich der Fraktur auf den jeweiligen Knochen aufgelegt und an den miteinander zu verbindenden Knochenfragmenten jeweils befestigt wird. Eine Befestigung wird dabei zumeist über Schrauben vorgenommen, wozu an der jeweiligen Osteosyntheseplatte dann mehrere Durchgangsöffnungen ausgebildet sind.

Bei derartigen Osteosyntheseplatten kommen verschiedene biokompatible Werkstoffe zum Einsatz, beispielsweise Titan, Titanlegierungen, Edelstahl oder biokompatible Polymere wie Polyetheretherketon (PEEK). Bei der Auswahl des Werkstoffs ist neben der erforderlichen Festigkeit und Biokompatibilität das Zusammenwirken mit den Befestigungsschrauben von großer Bedeutung. So lehrt beispielsweise die EP 2 956 073 A1 eine Druckplatte aus Reintitan zwischen Grade 0 und Grade 3, sodass die Druckplatte aus einem weicheren Werkstoff gebildet ist als eine damit zusammenwirkende Knochenschraube aus der Titanlegierung TiAl6V4. Durch eine derartige Werkstoffkombination kann die Knochenschraube die Druckplatte verformen, sodass ein Eingriff zwischen Schraubenkopf und Druckplatte verbessert wird. Ist eine höhere Stabilität der Druckplatte gefordert, so kann die Druckplatte ebenso wie die Knochenschraube aus der Titanlegierung TiAl6V4 gefertigt sein, wobei im Druckplattenkörper Inlays aus Reintitan zur Aufnahme der Knochenschrauben eingesetzt sind.

Das prozesssichere Einlegen von derartigen Inlays in eine Grundplatte ist allerdings aufwändig. Denn das Inlay darf sich beim Verschrauben der Grundplatte nicht verdrehen, sodass eine Verdrehsicherung erforderlich ist. Auch ein Herausdrücken des Inlays in Schraubrichtung aus der Grundplatte ist durch geeignete konstruktive Auslegung zu vermeiden.

Es ist daher eine erste Aufgabe der Erfindung, eine Osteosyntheseplatte bereitzustellen, welche sich durch eine hohe Stabilität auszeichnet und dennoch ein Einformen eines Gewindes beim Verschrauben ermöglicht, und dabei einfach und prozesssicher herzustellen ist. Es eine weitere Aufgabe der Erfindung, ein Verfahren zur Herstellung einer derartigen Osteosyntheseplatte anzugeben.

Die erste Aufgabe wird gelöst durch die Merkmale des Patentanspruchs 1. Die weitere Aufgabe wird gelöst durch die Merkmale des Patentanspruchs 19. Vorteilhafte Ausgestaltungen ergeben sich aus den abhängigen Ansprüchen, der Beschreibung sowie aus den Figuren.

Zur Lösung der ersten Aufgabe wird eine Osteosyntheseplatte zur Fixierung von Knochenfragmenten vorgeschlagen, welche einen Grundkörper mit einer Oberseite und mit einer der Oberseite gegenüberliegenden Unterseite aufweist. Die Unterseite ist zur Auflage der Osteosyntheseplatte auf den Knochenfragmenten vorgesehen. Die Osteosyntheseplatte weist mehrere Öffnungen auf, welche sich zwischen der Oberseite und der Unterseite erstrecken. Durch Einschrauben von je einer Schraube durch eine derartige Öffnung kann die Osteosyntheseplatte am Knochen befestigt werden.

Erfindungsgemäß ist vorgesehen, dass der Grundkörper einen mehrschichtigen Aufbau aufweist, umfassend eine Kernschicht, eine die Kernschicht zumindest teilweise bedeckende erste Deckschicht, und eine die Kernschicht zumindest teilweise bedeckende zweite Deckschicht. Die erste Deckschicht ist auf einer ersten Seite der Kernschicht angeordnet. Die zweite Deckschicht ist auf einer zweiten Seite der Kernschicht angeordnet, welcher der ersten Seite gegenüberliegt. Die Kernschicht ist dabei aus einem ersten Werkstoff gefertigt, während die beiden Deckschichten aus einem zweiten Werkstoff gebildet sind, welcher sich vom ersten Werkstoff unterscheidet. Der erste Werkstoff weist eine höhere Duktilität auf als der zweite Werkstoff. In anderen Worten ist der Widerstand der Kernschicht gegen plastisches Verformen geringer als der Widerstand der Deckschichten gegen plastisches Verformen. Unter einer "höheren Duktilität" wird im Rahmen der gegenständlichen Unterlagen vorzugsweise verstanden, dass die Zugfestigkeit des weniger-duktilen Werkstoffs um mindestens den Faktor 1,5 größer ist als die Zugfestigkeit des höher-duktilen Werkstoffs.

Durch den erfindungsgemäßen Aufbau weist die Osteosyntheseplatte einen verhältnismäßig weichen, duktilen Kern auf, während die Deckschichten aus einem im Vergleich zum Kern härteren, weniger duktilen Material gebildet sind. Dadurch wird bei einer Biegebeanspruchung der Osteosyntheseplatte im am Knochen verschraubten Zustand eine hohe Stabilität gewährleistet, ohne auf die Verschraubungs-Vorteile des weichen Kerns verzichten zu müssen. Aufgrund des Schichtaufbaus ist bei der Herstellung der Osteosyntheseplatte kein aufwändiges Herstellen von Fügebereichen zum Einlegen von Inlays erforderlich. Auch eine Verdrehsicherung und eine Sicherung der Inlays gegen axiales Herausdrücken kann entfallen.

Gemäß einer ersten möglichen Variante ist die Kernschicht aus Reintitan gebildet, beispielsweise aus Titan Grade 1, Titan Grade 2, Titan Grade 3 oder Titan Grade 4, wobei Titan Grade 2 besonders bevorzugt ist. Die erste und zweite Deckschicht ist in dieser ersten Variante aus einer Titanlegierung gebildet, beispielsweise aus Ti6Al4V oder aus TiAl6Nb7. Alternativ dazu kann die erste und zweite Deckschicht in der ersten Variante aus Stahl gebildet sein, insbesondere aus einer rost- und säurebeständigen Stahlsorte, beispielsweise aus X2CrNiMo18-15-3, oder aus X2CrMnMo 18-13-3, oder aus X4CrNiMnMo 21-9-4 oder aus CoCr28Mo6 mit einem Kohlenstoffgehalt größer 0,2 %.

Zur Befestigung einer Osteosyntheseplatte gemäß dieser ersten möglichen Variante am Knochen werden vorzugsweise Schrauben aus Ti6Al4V, aus X2CrNiMo18-15-3 oder aus X2CrMnMo 18-13-3 eingesetzt. Diese Schraubenwerkstoffe weisen eine höhere Festigkeit als die Kernschicht auf, sodass die Schrauben ein Gewinde in die Kernschicht einformen können, oder ein in der Kernschicht bestehendes Gewinde verformen können. Dadurch kann der Halt der Schraube verbessert werden. Durch Kombination von einer Osteosyntheseplatte und mehreren Schrauben kann ein Set gebildet werden.

Gemäß einer zweiten möglichen Variante ist die Kernschicht aus einer ersten Stahlsorte gebildet, während die erste und zweite Deckschicht aus einer zweiten Stahlsorte oder aus einer Titanlegierung gebildet ist. Die erste Stahlsorte gemäß der zweiten Variante kann beispielsweise aus CoCr28Mo6 mit einem Kohlenstoffgehalt kleiner 0,055 % oder aus X2CrNiMo18-15-3 oder aus X4CrNiMnMo 21-9-4 oder aus X2CrMnMo 18-13-3 gebildet sein. Die zweite Stahlsorte gemäß der zweiten Variante kann aus CoCr28Mo6 mit einem Kohlenstoffgehalt größer 0,2 % oder aus X2CrNiMo18-15-3 oder aus X4CrNiMnMo 21-9-4 oder aus X2CrMnMo 18-13-3 gebildet sein. Die Titanlegierung für die Deckschichten gemäß der zweiten Variante kann beispielsweise aus TiAl6Nb7 oder aus Ti6Al4V gebildet sein. Hierbei ist zu beachten, dass die Zugfestigkeit dieser Werkstoffe davon abhängt, ob sie im kaltverfestigten oder geglühten Zustand vorliegen. Bei der jeweiligen Kombination der Werkstoffe für Kernschicht und Deckschichten ist daher darauf zu achten, dass sie im fertigen Zustand der Osteosyntheseplatte einen geeigneten Duktilitätsunterschied aufweisen.

Zur Befestigung einer Osteosyntheseplatte gemäß dieser zweiten möglichen Variante am Knochen sind vorzugsweise Schrauben aus folgenden Werkstoffen zu wählen:
- Ti6Al4V, X2CrNiMo18-15-3 oder X2CrMnMo 18-13-3 bei einer Kernschicht der Osteosyntheseplatte aus CoCr28Mo6 mit einem Kohlenstoffgehalt kleiner 0,055 %;
- X2CrMnMo 18-13-3 bei einer Kernschicht der Osteosyntheseplatte aus X2CrNiMo18-15-3;
- X2CrNiMo18-15-3 bei einer Kernschicht der Osteosyntheseplatte aus X2CrMnMo 18-13-3.

Diese Schraubenwerkstoffe weisen bei geeigneter Wärmebehandlung und/oder Kaltverfestigung eine höhere Festigkeit als die Kernschicht auf, sodass die Schrauben ein Gewinde in die Kernschicht einformen können, oder ein in der Kernschicht bestehendes Gewinde verformen können. Dadurch kann der Halt der Schraube verbessert werden. Durch Kombination von einer Osteosyntheseplatte und mehreren Schrauben kann ein Set gebildet werden.

Vorzugsweise ist in zumindest einer der Öffnungen eine Schulter zur Abstützung der Schraube ausgebildet, wobei diese Schulter in der Kernschicht angeordnet ist. Durch eine derartige Ausgestaltung kann sich die Schraube in der Kernschicht abstützen und dabei ein Gewinde in der Kernschicht einformen. Dies verbessert den Halt der Schraube und bietet eine größtmögliche Freiheit bei der Wahl des Winkels zwischen Schraube und Osteosyntheseplatte.

Vorzugsweise ist in zumindest einer der Öffnungen ein Innengewinde ausgebildet. Das Innengewinde ist dabei entweder ausschließlich oder zu einem überwiegenden Anteil in der Kernschicht ausgebildet und ist dazu eingerichtet, mit einem Außengewinde der Schraube zusammenzuwirken. Ist das Innengewinde nicht vollständig in der Kernschicht ausgebildet, so ist vorzugsweise nur ein kurzer Einlauf oder Auslauf eines Gewindegangs in einer der Deckschichten angeordnet. Vollständige Gewindegänge sind ausschließlich in der Kernschicht angeordnet.

Vorzugsweise weist die Kernschicht, die erste Deckschicht und/oder die zweite Deckschicht eine konstante Schichtdicke auf. Besonders bevorzugt ist eine Ausführung, bei der die erste Deckschicht die gleiche Schichtdicke wie die zweite Deckschicht aufweist. Eine solche Ausführung ist besonders dann vorteilhaft, wenn die Deckschichten bei der Fertigung der Osteosyntheseplatte nicht abgetragen werden sollen, um die gewünschte Schichtdicke zu erreichen. Denn durch einen derartigen symmetrischen Schichtaufbau kann ein versehentliches Verwechseln von Unterseite und Oberseite bei der Herstellung ausgeschlossen werden.

Alternativ dazu kann vorgesehen sein, dass die erste Deckschicht und/oder die zweite Deckschicht eine nicht-konstante Schichtdicke aufweisen. Eine derartige Ausgestaltung kann beispielsweise dann vorteilhaft sein, wenn die Osteosyntheseplatte in eine Biegerichtung eine deutlich andere Belastung aufweist als in die andere Biegerichtung, sodass eine belastungsoptimierte Geometrie gewählt werden kann. Eine derartige Ausgestaltung ist auch dann vorteilhaft, wenn die Osteosyntheseplatte an einem bestimmten Abschnitt durch den Chirurgen plastisch verformt werden soll. In diesem Abschnitt kann die Osteosyntheseplatte eine geringere Schichtdicke der Deckschicht aufweisen, oder lokal unbeschichtet sein.

Gemäß einer ersten möglichen Ausgestaltung kann die erste Deckschicht ausschließlich an der Oberseite, und die zweite Deckschicht ausschließlich an der Unterseite der Osteosyntheseplatte ausgebildet sein. Denn im implantierten Zustand weist die Osteosyntheseplatte an der Ober- und Unterseite in zahlreichen Anwendungsfällen die höchsten Zug- und Druckspannungen auf, sodass durch die Ausbildung der im Vergleich zur Kernschicht härteren Deckschichten an der Ober- und Unterseite eine belastungsoptimierte Geometrie gebildet wird.

Vorzugsweise bedeckt die erste Deckschicht und/oder die zweite Deckschicht die Kernschicht an der Oberseite bzw. an der Unterseite der Osteosyntheseplatte vollflächig.

Von einer derartigen vollflächigen Bedeckung sind Öffnungen zwischen Oberseite und Unterseite, sowie einseitig ausgebildete Aufnahme- und Positionierungs-Geometrien wie beispielsweise Kugelkalotten oder Sacklöcher ausgenommen. Auch Fasen an den Übergängen zwischen den Flächen sowie Nahtlöcher an den Rändern sind hiervon ausgenommen. Unter einer vollflächigen Ausbildung der Deckschicht an der Ober- bzw. Unterseite wird demnach verstanden, dass glatte, nicht funktionsbildende Flächen der Ober, bzw. Unterseite von der ersten, bzw. zweiten Deckschicht bedeckt sind.

Bei einer Ausbildung der ersten und zweiten Deckschicht an der Oberseite, bzw. an der Unterseite der Osteosyntheseplatte beträgt die Schichtdicke der ersten und zweiten Deckschicht vorzugsweise zwischen 0,2 und 2 Millimeter, besonders bevorzugt zwischen 0,2 und 1 Millimeter. Eine derartige Dimensionierung der Deckschichten hat sich als vorteilhaft herausgestellt.

Gemäß einer weiteren möglichen Ausführung bedeckt die erste und/oder die zweite Deckschicht die Kernschicht nur abschnittsweise, sodass die Kernschicht an der Oberseite, bzw. an der Unterseite teilweise freiliegt. Eine derartige Ausgestaltung ist vorteilhaft, wenn die Osteosyntheseplatte an einem bestimmten Abschnitt durch den Chirurgen plastisch verformt werden soll. Weist die Osteosyntheseplatte beispielsweise einen Aufbau mit einem Kopfbereich und einem Schaftbereich auf, wobei der Kopfbereich dazu eingerichtet ist an einer Metaphyse befestigt zu werden, und der Schaftbereich dazu eingerichtet ist an einer Diaphyse befestigt zu werden, so könnte der Schaftbereich zumindest abschnittsweise ohne Deckschicht ausgebildet werden, um die Osteosyntheseplatte in diesem Bereich einfacher an die individuelle Anatomie des Patienten anpassen zu können.

Vorzugsweise weist die Kernschicht eine zumindest 1,5 mal, besonders bevorzugt dreimal so hohe Schichtdicke auf wie die erste und zweite Deckschicht an ihrer dicksten Stelle. Durch eine solche Ausgestaltung wird eine Osteosyntheseplatte bereitgestellt, welche an der Oberseite und Unterseite eine dünne und vergleichsweise harte Schicht aufweist, welche die hohen Zugspannungen, bzw. Druckspannungen ohne plastische Verformung tragen kann, und einen dicken und vergleichsweise weichen Kern aufweist, welcher für das Einformen eines Gewindes gut geeignet ist. Besonders bevorzugte Verhältnisse der Schichtdicken von Kernschicht und Deckschichten sind: ein Zehntel der Gesamtdicke der Osteosyntheseplatte als Schichtdicke jeder einzelnen Deckschicht und acht Zehntel der Gesamtdicke der Osteosyntheseplatte als Schichtdicke der Kernschicht; oder ein Zwanzigstel der Gesamtdicke der Osteosyntheseplatte als Schichtdicke jeder einzelnen Deckschicht und achtzehn Zwanzigstel der Gesamtdicke der Osteosyntheseplatte als Schichtdicke der Kernschicht.

Bei einer derartigen Ausgestaltung kann die in der Kernschicht angeordnete Schulter zur Abstützung der Schraube schräg zu einer Erstreckungsrichtung der Kernschicht angeordnet sein. Dadurch kann eine Vorausrichtung der Schraube ermöglicht werden, welche von einer orthogonalen Richtung ausgehend von der Unterseite der Osteosyntheseplatte abweicht. Durch eine solche Gestaltung kann die Osteosyntheseplatte für Frakturen verwendet werden, bei denen eine derartige Vorausrichtung von hoher Bedeutung ist, wie beispielsweise Frakturen im Bereich der Metaphyse oder der Epiphyse eines Röhrenknochens.

Gemäß einer alternativen Ausgestaltung weist die Kernschicht entweder eine geringere oder eine maximal 1,5 mal so hohe Schichtdicke auf wie die erste und zweite Deckschicht an ihrer dicksten Stelle. Als beispielhafte Grenze für eine Kernschicht mit geringerer Schichtdicke als die Deckschichten kann ein Verhältnis angesehen werden, bei dem die Kernschicht eine Schichtdicke von einem Fünftel der Gesamtdicke der Osteosyntheseplatte aufweist, und jede der Deckschichten eine Schichtdicke von zwei Fünftel der Gesamtdicke der Osteosyntheseplatte aufweist. Eine solche Ausgestaltung ist besonders bei dünnwandigen Platten vorteilhaft, wie sie beispielsweise in der Mund-, Kiefer- und Gesichtschirurgie, bei Rippenfrakturen oder bei Frakturen von Beckenknochen zum Einsatz kommen. Eine solche Ausgestaltung eignet sich besonders in einer Ausführung mit konstanter Schichtdicke der Kernschicht und der beiden Deckschichten.

Bei einer derartigen Ausgestaltung ist die in der Kernschicht angeordnete Schulter zur Abstützung der Schraube vorzugsweise parallel zu einer Erstreckungsrichtung der Kernschicht angeordnet. Eine Vorausrichtung der Schraubaufnahme, welche von einer orthogonalen Richtung ausgehend von der Unterseite der Osteosyntheseplatte abweicht, ist bei einer derartigen Gestaltung somit nicht gegeben. Dies ist bei dünnwandigen Osteosyntheseplatten jedoch häufig nicht erforderlich. Somit kann das Einformen des Gewindes auch bei einer derartigen Ausgestaltung ausschließlich oder zu einem überwiegenden Anteil in der Kernschicht erfolgen, sodass ein zuverlässiger Halt der Schraube gewährleistet wird.

Gemäß einer weiteren möglichen Ausgestaltung kann sich die erste Deckschicht und die zweite Deckschicht entlang von gegenüberliegenden Seitenwänden der Osteosyntheseplatte erstrecken, sodass sich die Kernschicht, die erste Deckschicht und die zweite Deckschicht von der Oberseite bis zur Unterseite erstrecken. In anderen Worten sind die erste und zweite Deckschicht in dieser Ausgestaltung an den Seitenrändern ausgebildet, und nicht an der Ober- und Unterseite der Osteosyntheseplatte. Bei einer derartigen Ausgestaltung beträgt die Schichtdicke der ersten und/oder zweiten Deckschicht vorzugsweise zwischen 0,5 und 5 Millimeter.

Vorzugsweise weist die Osteosyntheseplatte eine längliche, nicht-plane Form auf. Durch eine derartige Gestaltung ist die Osteosyntheseplatte besonders gut an die Anatomie des Knochens angepasst. Der schichtartige Aufbau kann auch bei einer derartigen nicht-planen Form angewendet werden, wie anhand der nachfolgenden Ausführungen zu den Herstellungsverfahren deutlich gemacht wird.

Zur Lösung der zweiten Aufgabe wird ein Verfahren zur Herstellung einer Osteosyntheseplatte mit den folgenden Schritten vorgeschlagen. In einem ersten Schritt wird ein planes Rohmaterial bereitgestellt, welches die Kernschicht sowie die erste und zweite Deckschicht aufweist. Ein derartiges Rohmaterial kann auf verschiedene Weisen hergestellt werden, beispielsweise durch Walzen oder Walzplattieren. In einem zweiten Schritt wird eine Zwischenstufe der Osteosyntheseplatte aus dem Rohmaterial herausgetrennt. In einem dritten Schritt findet eine Umformung statt, um eine gewünschte nicht-plane Form der Osteosyntheseplatte zu erzielen. Die nicht-plane Form wird anschließend in einem vierten Schritt wärmebehandelt. In einem optionalen zusätzlichen Schritt findet eine Oberflächenbearbeitung und/oder eine Oberflächenbeschichtung der wärmebehandelten Osteosyntheseplatte statt.

Das im zweiten Schritt durchgeführte Heraustrennen aus dem Rohmaterial kann auf verschiedene Weisen erfolgen, beispielsweise durch Wasserstrahlschneiden, Laserschneiden, Stanzen oder Fräsen. Das im dritten Schritt durchgeführte Umformen kann ebenfalls auf verschiedene Weisen durchgeführt werden, beispielsweise durch Kaltumformen oder Warmumformen. Die optional durchgeführte Oberflächenbearbeitung kann beispielsweise durch eine spanende Bearbeitung oder durch Strahlen erfolgen. Die optionale Oberflächenbeschichtung kann beispielsweise ein Anodisieren sein.

Die im vierten Schritt durchgeführte Wärmebehandlung kann beispielsweise ein Lösungsglühen, ein Ausscheidungsglühen oder ein Rekristallationsglühen sein. Gemäß einer ersten möglichen Ausführungsvariante kann im vierten Schritt ein Lösungsglühen und/oder ein Rekristallationsglühen bei einer Temperatur bis zu 900 Grad Celsius durchgeführt werden, beispielsweise unter einem Argon-Schutzgas. Anschließend erfolgt eine Wasserabkühlung und/oder ein Presshärten, sowie ein Auslagern bei etwa 500 Grad Celsius für eine Zeitdauer von vier bis acht Stunden. Gemäß einer zweiten möglichen Ausführungsvariante erfolgt ein Lösungsglühen bei einer Temperatur von etwa 1020 Grad Celsius, beispielsweise unter einem Argon-Schutzgas. Anschließend erfolgt eine einfache Abkühlung. Ein auf das Lösungsglühen anschließendes Auslagern ist in dieser zweiten Ausführungsvariante nicht erforderlich.

Vorzugsweise wird der zweite Schritt (Trennen) vor dem dritten Schritt (Umformen) durchgeführt, sodass die aus dem Rohmaterial getrennte Zwischenstufe eine plane Form aufweist. Alternativ dazu ist auch denkbar, dass der zweite Schritt nach dem dritten Schritt durchgeführt wird, sodass die aus dem Rohmaterial getrennte Zwischenstufe bereits die gewünschte nicht-plane Form aufweist. Durch die nicht-plane Form ist die Osteosyntheseplatte besonders gut an die Anatomie des Knochens angepasst, sodass eine weitere Umformung durch den Chirurgen während der operativen Anbringung der Osteosyntheseplatte an den Knochen entweder nicht oder nur zu einem geringem Maß erforderlich ist.

Vorzugsweise umfasst das Herstellungsverfahren in einem fünften Schritt eine spanende Bearbeitung zur Herstellung einer Geometrie der Öffnungen der Osteosyntheseplatte. In diesem fünften Schritt können neben den Öffnungen auch weitere Geometrien hergestellt werden, beispielsweise Nahtanker oder Gewinde zur Befestigung eines Bohrblocks auf der Osteosyntheseplatte. Der fünfte Schritt wird entweder unmittelbar nach dem zweiten Schritt (Trennen), nach dem dritten Schritt (Umformen) oder nach dem vierten Schritt (Wärmbehandeln) durchgeführt.

Die beschriebenen Schritte des Herstellungsverfahrens sind nicht abschließend, sodass weitere Herstellungsschritte enthalten sein können. Beispielsweise kann ein Kantenbrechen, Entgraten oder Schleifen zur Herstellung einer für die chirurgische Anwendung geeignete Oberfläche vorgesehen sein. Der Übersichtlichkeit halber sind diese Schritte nicht explizit genannt, da ein Fachmann derartige Schritte an der geeigneten Stelle des Herstellungsverfahrens einsetzen wird.

Ausführungsbeispiele der Erfindung sind anhand der Figuren detailliert beschrieben. Es zeigen:
- Fig. 1 und Fig. 2: je eine Ansicht einer Osteosyntheseplatte gemäß einem ersten Ausführungsbeispiel;
- Fig. 3 und Fig. 4: je eine Schnittansicht der Osteosyntheseplatte gemäß dem ersten Ausführungsbeispiel;
- Fig. 5: eine Schnittansicht einer Osteosyntheseplatte gemäß einem zweiten Ausführungsbeispiel;
- Fig. 6 und Fig. 7: je eine Ansicht einer Osteosyntheseplatte gemäß einem dritten Ausführungsbeispiel;
- Fig. 8 bis Fig. 13: verschiedene Ansichten von Zwischenstufen bei der Herstellung einer Osteosyntheseplatte gemäß dem ersten Ausführungsbeispiel,
- Fig. 14 bis Fig. 22: verschiedene Ansichten von Zwischenstufen bei der Herstellung einer Osteosyntheseplatte gemäß dem dritten Ausführungsbeispiel,
- Fig. 23: eine Ansicht einer Osteosyntheseplatte mit darin angeordneten Schrauben;
- Fig. 24 bis Fig. 26: je eine Ansicht einer Osteosyntheseplatte gemäß einem vierten Ausführungsbeispiel;
- Fig. 27 bis Fig. 29: je eine Ansicht einer Osteosyntheseplatte gemäß einem fünften Ausführungsbeispiel;
- Fig. 30: eine Ansicht einer Osteosyntheseplatte gemäß einem sechsten Ausführungsbeispiel;
- Fig. 31 und Fig. 32: je eine Ansicht einer Osteosyntheseplatte gemäß einem siebenten Ausführungsbeispiel;
- Fig. 33 und Fig. 34: je eine Ansicht einer Osteosyntheseplatte gemäß einem achten Ausführungsbeispiel; und
- Fig. 35 bis Fig. 37: je eine Ansicht einer Osteosyntheseplatte gemäß einem neunten Ausführungsbeispiel.

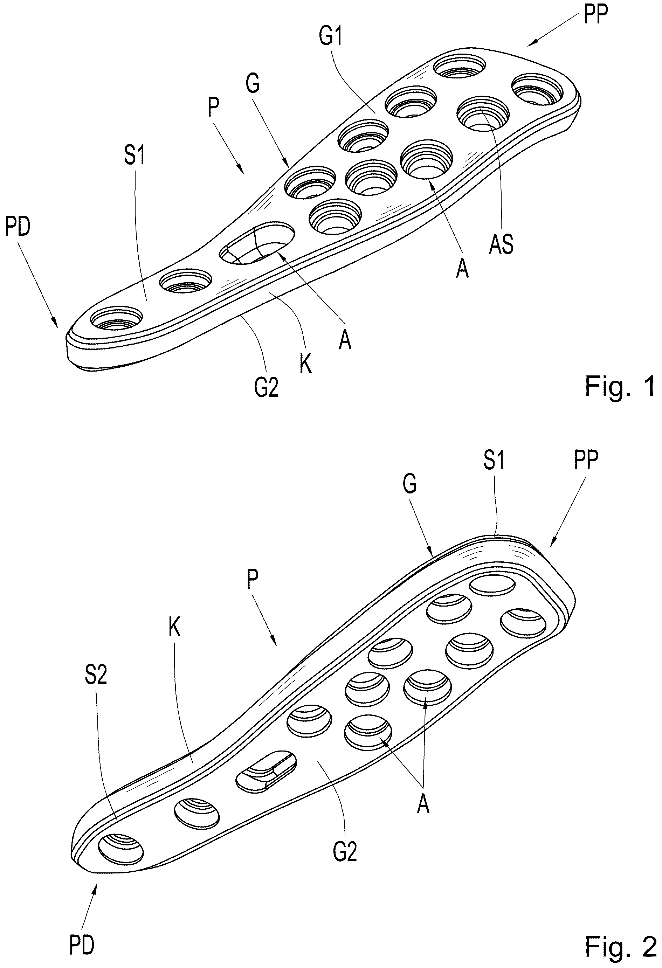

Fig. 1 und Fig. 2 zeigen je eine isometrische Ansicht einer Osteosyntheseplatte P gemäß einem ersten Ausführungsbeispiel. Die Osteosyntheseplatte P weist einen Grundkörper G mit einer Oberseite G1 und mit einer Unterseite G2 auf. Die Unterseite G2 ist zur Auflage der Osteosyntheseplatte P auf in den Figuren nicht dargestellten Knochenfragmenten vorgesehen. Die Osteosyntheseplatte P weist mehrere Öffnungen A auf, welche die Osteosyntheseplatte P von der Oberseite G1 zur Unterseite G2 durchdringen. Die Öffnungen A sind dazu eingerichtet, je eine in Fig. 1 und Fig. 2 nicht dargestellte Schraube aufzunehmen. Mittels der Schrauben wird die Osteosyntheseplatte P auf den Knochenfragmenten befestigt. Dazu weist zumindest eine der Öffnungen A eine Schulter AS auf, auf die ein Abschnitt der Schraube abgestützt werden kann. Das in Fig. 1 und Fig. 2 dargestellte Ausführungsbeispiel zeigt eine Osteosyntheseplatte P für die Anwendung am proximalen Humerus, mit einem proximalen Ende PP und einem distalen Ende PD. Die Osteosyntheseplatte P weist eine längliche und gewölbte, nicht-plane Form auf, um bestmöglich an die Anatomie eines Knochens angepasst zu sein, hier beispielhaft für die Form des proximalen Humerus.

Der Grundkörper G der Osteosyntheseplatte P weist einen mehrschichtigen Aufbau auf, welcher eine Kernschicht K sowie eine erste und zweite Deckschicht S1, S2 umfasst. Die erste Deckschicht S1 bedeckt die Kernschicht K an der Oberseite G1. Die zweite Deckschicht S2 bedeckt die Kernschicht K an der Unterseite G2. Die Kernschicht K ist aus Reintitan gebildet, beispielsweise aus Titan Grade 2. Die erste und zweite Deckschicht S1, S2 ist aus einer Titanlegierung gebildet, beispielsweise aus Ti6Al4V. Alternativ dazu könnte die erste und zweite Deckschicht S1, S2 aus Stahl gebildet sein, beispielsweise aus X2CrNiMo18-15-3 oder aus X4CrNiMnMo 21-9-4. Die erste und zweite Deckschicht S1, S2 weisen im ersten Ausführungsbeispiel eine gleiche, konstante Schichtdicke auf, welche im vorliegenden Beispiel etwa 0,4 mm beträgt. Die Kernschicht K ist im Vergleich zu den Deckschichten S1, S2 deutlich dicker, und weist im vorliegenden Beispiel eine Dicke von etwa 3,2 mm auf.

Gemäß einer alternativen Ausgestaltung ist die Kernschicht K aus der Stahlsorte CoCr28Mo6 mit einem Kohlenstoffgehalt kleiner 0,055 % gebildet, während die beiden Deckschichten S1, S2 aus der Stahlsorte CoCr28Mo6 mit einem Kohlenstoffgehalt größer 0,2 % gebildet sind. Gemäß einer zweiten alternativen Ausgestaltung ist die Kernschicht K aus der Stahlsorte CoCr28Mo6 mit einem Kohlenstoffgehalt kleiner 0,055 % gebildet, während die beiden Deckschichten S1, S2 aus der Titanlegierung TiAl6Nb7 oder aus der Titanlegierung Ti6Al4V gebildet sind, wobei die verwendete Titanlegierung im kaltverfestigten Zustand vorliegt. Gemäß einer dritten alternativen Ausgestaltung ist die Kernschicht K aus der Stahlsorte CoCr28Mo6 mit einem Kohlenstoffgehalt kleiner 0,055 % gebildet, während die beiden Deckschichten S1, S2 aus der Stahlsorte X2CrMnMo 18-13-3 gebildet sind. Gemäß einer vierten alternativen Ausgestaltung ist die Kernschicht K aus der Stahlsorte X2CrNiMo18-15-3 gebildet, während die Deckschichten S1, S2 aus X2CrMnMo 18-13-3 gebildet sind.

Die vorliegende Erfindung ist nicht auf Osteosyntheseplatten P für den proximalen Humerus beschränkt. Vielmehr lässt sich der erfindungsgemäße mehrschichtige Aufbau bei verschiedenen Osteosyntheseplatten P anwenden, wie beispielsweise für die Anwendung am distalen Radius, am Femur, am Cranium oder am Thorax. Auch die im ersten Ausführungsbeispiel ausgeführte Dicke der Kernschicht K und der Deckschichten S1, S2 ist nur beispielhaft anzusehen.

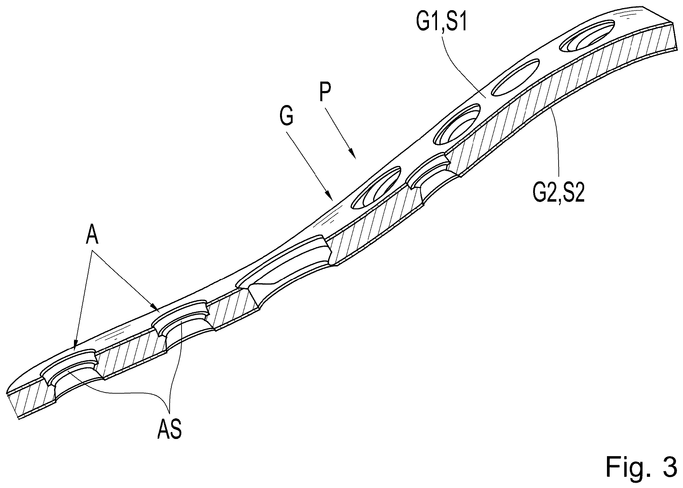

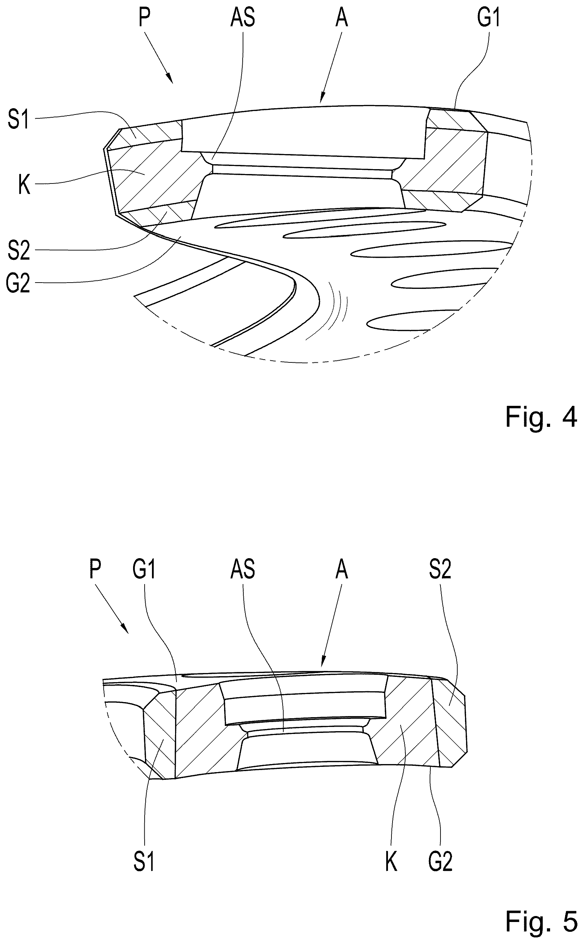

Fig. 3 und Fig. 4 zeigen je eine Schnittansicht eines Teils der Osteosyntheseplatte P gemäß dem ersten Ausführungsbeispiel. In Fig. 3 ist ein Schnitt entlang einer Längserstreckung der Osteosyntheseplatte P dargestellt. In Fig. 4 ist ein Schnitt quer zur Längserstreckung der Osteosyntheseplatte P dargestellt. In beiden Darstellungen ist deutlich zu erkennen, dass die Schulter AS einer der Öffnungen A vollständig in der Kernschicht K angeordnet ist. Die Anordnung der Schulter AS kann, wie in Fig. 4 deutlich zu erkennen, schräg zu einer Erstreckungsrichtung der Kernschicht K angeordnet sein, sodass eine gewünschte Vorausrichtung einer Schraube erzielt werden kann. Die in Fig. 4 dargestellte Geometrie der Schulter AS ist nur beispielhaft anzusehen. Zur Klarstellung sei festgehalten, dass nicht in jeder Öffnung der Osteosyntheseplatte P zwischen der Oberseite G1 und der Unterseite G2 eine derartige Schulter angeordnet sein muss.

Fig. 5 zeigt einen Schnitt durch eine Osteosyntheseplatte P gemäß einem zweiten Ausführungsbeispiel. Im Unterschied zu dem in Fig. 1 bis 4 dargestellten ersten Ausführungsbeispiel erstrecken sich die Deckschichten S1, S2 entlang von gegenüberliegenden Seitenwänden der Osteosyntheseplatte P. Die Kernschicht K sowie die beiden Deckschichten S1, S2 erstrecken sich dabei von der Oberseite G1 bis zur Unterseite G2. Die Schulter AS der Öffnung A ist auch bei dieser Ausführung vollständig in der Kernschicht K angeordnet. Die Schichtdicke der Deckschichten S1, S2 beträgt bei dieser Ausführung etwa 3 mm, gemessen von der jeweiligen Seitenwand der Osteosyntheseplatte P bis zur Kernschicht K.

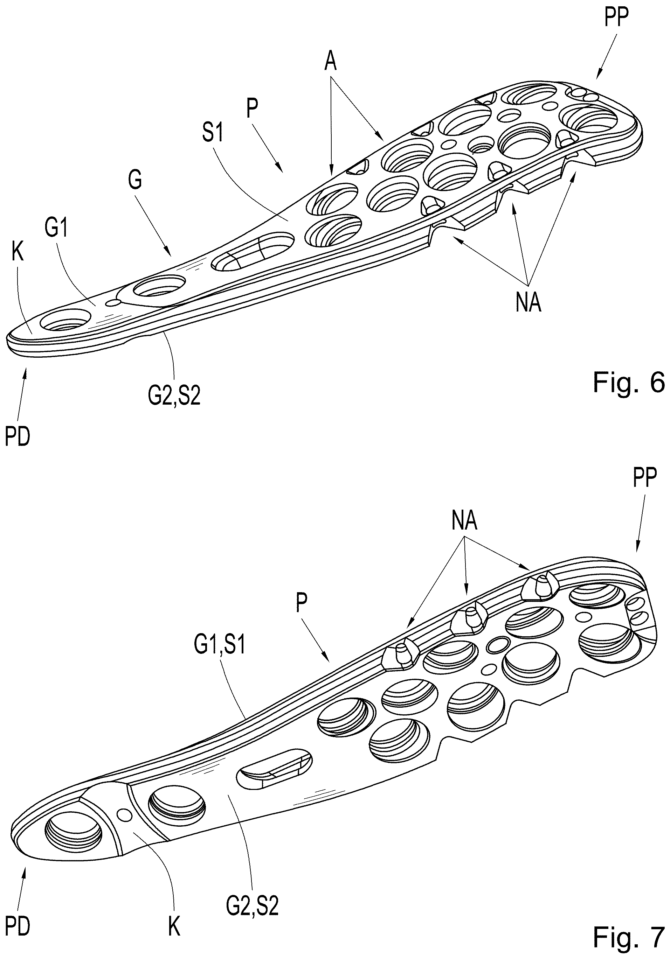

Fig. 6 und Fig. 7 zeigen je eine isometrische Ansicht einer Osteosyntheseplatte P gemäß einem dritten Ausführungsbeispiel. Im Unterschied zu dem in Fig. 1 bis 4 dargestellten ersten Ausführungsbeispiel weisen die Deckschichten S1, S2, ausgebildet an Oberseite G1 und Unterseite G2, eine nicht-konstante Schichtdicke auf. Im dargestellten Ausführungsbeispiel liegt die Kernschicht K im Bereich des distalen Endes PD an der Oberseite G1 und an der Unterseite G2 zumindest teilweise frei. In anderen Worten sind die Oberseite G1 und die Unterseite G2 der Osteosyntheseplatte P gemäß dem dritten Ausführungsbeispiel nicht vollflächig von der ersten, bzw. zweiten Deckschicht S1, S2 bedeckt. Dadurch kann der distale Bereich PD einfacher plastisch verformt werden, um die Osteosyntheseplatte P an dieser Stelle bestmöglich an die individuelle Anatomie anpassen zu können. Die lokal reduzierte Dicke der Deckschichten S1, S2 bzw. das lokale Freiliegen der Kernschicht K an der Oberseite G1 und Unterseite G2 erfolgt durch entsprechende spanende Bearbeitung an diesen Stellen.

An den Seitenrändern des proximalen Abschnitts PP sind mehrere Nahtanker NA ausgebildet, beispielsweise um Sehnen oder Muskeln an der Osteosyntheseplatte P annähen zu können. Die Nahtanker NA werden durch Öffnungen zwischen der Oberseite G1 und der Unterseite G2 gebildet. Diese Öffnungen sind in der Ausführung gemäß dem dritten Ausführungsbeispiel ausschließlich in der ersten Deckschicht S1 angeordnet. In der Kernschicht K und in der zweiten Deckschicht S2 sind Freistellungen angeordnet, um die Nahtanker NA besser zugänglich zu machen, wenn die Osteosyntheseplatte mit der Unterseite G2 auf dem Knochen aufgelegt ist.

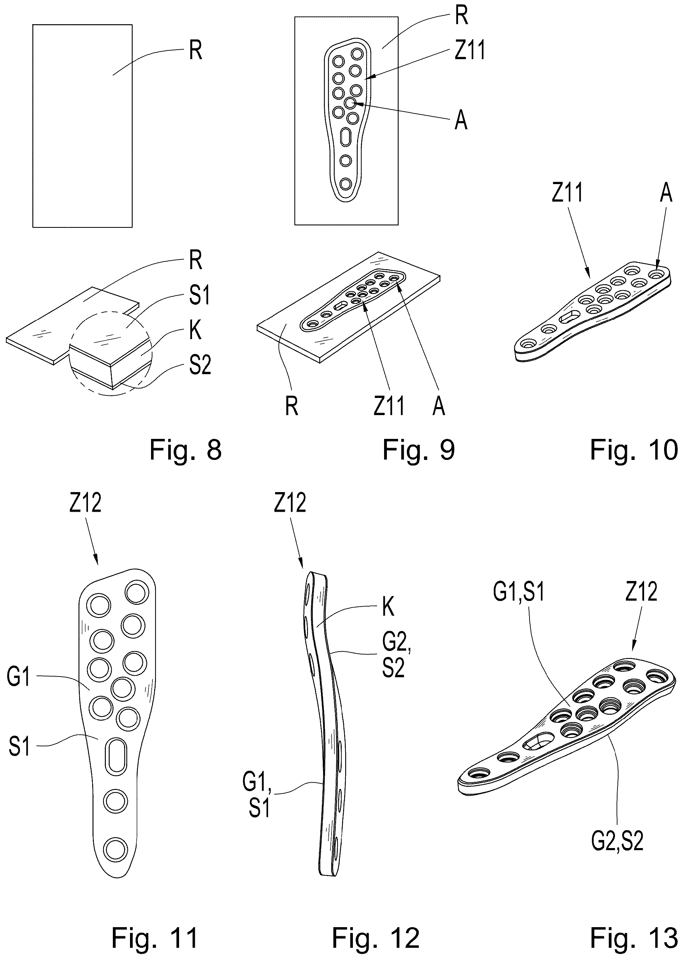

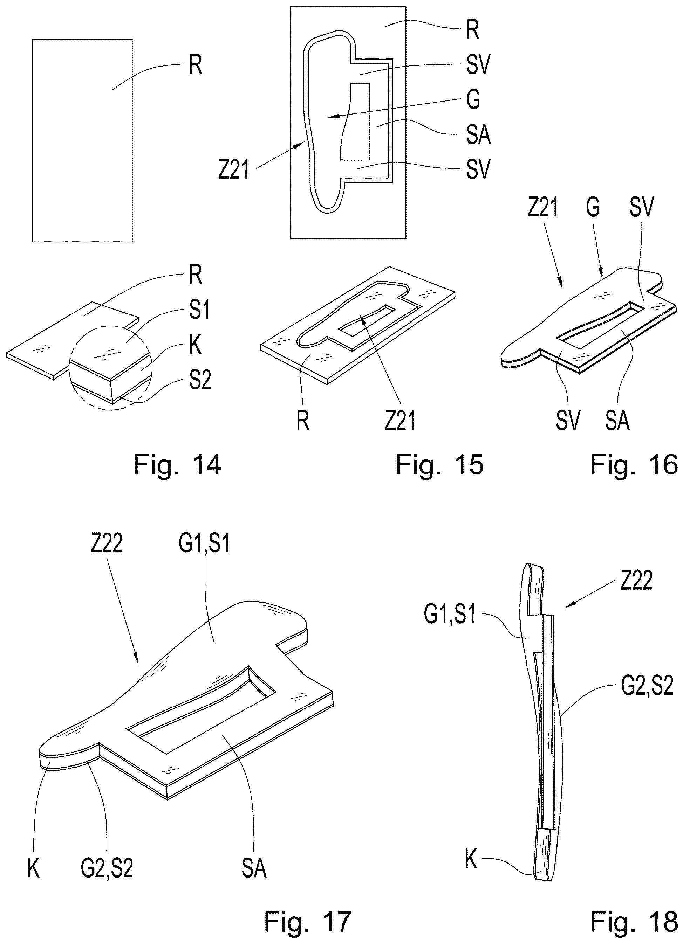

In Fig. 8 bis Fig. 22 werden verschiedene Verfahren zur Herstellung der Osteosyntheseplatte P beschrieben, indem Zwischenstufen der Osteosyntheseplatte P bei der Herstellung dargestellt sind. Dabei zeigen Fig. 8 bis Fig. 13 Zwischenstufen gemäß einem ersten Herstellungsverfahren, und Fig. 14 bis Fig. 22 Zwischenstufen gemäß einem zweiten Herstellungsverfahren.

Fig. 8 zeigt in der oberen Darstellung eine Draufsicht auf ein planes Rohmaterial R, und in der unteren Darstellung eine isometrische Ansicht des Rohmaterials R, wobei eine Ecke des Rohmaterials R in vergrößerter Darstellung wiedergegeben ist. Das Rohmaterial R weist bereits den mehrschichtigen Aufbau mit Kernschicht K sowie erster und zweiter Deckschicht S1, S2 auf. In einem ersten Verfahrensschritt zur Herstellung der Osteosyntheseplatte P wird ein derartiges Rohmaterial R bereitgestellt.

Fig. 9 zeigt in einen oberen Darstellung eine Draufsicht auf das plane Rohmaterial R, aus dem eine plane Zwischenstufe Z11 herausgetrennt wurde. Die untere Darstellung von Fig. 9 zeigt eine entsprechende isometrische Ansicht. In einem zweiten Verfahrensschritt zur Herstellung der Osteosyntheseplatte P erfolgt ein derartiges Trennen der Zwischenstufe Z11 aus dem Rohmaterial R. Das Heraustrennen der planen Zwischenstufe Z11 kann beispielsweise durch Wasserstrahlschneiden oder Laserschneiden erfolgen. Die plane Zwischenstufe Z11 weist bereits die Öffnungen A auf. Die Öffnungen A können in der Zwischenstufe Z11 bereits fertig vorliegen. Alternativ dazu können die Öffnungen A in der Zwischenstufe Z11 nur vorgeformt sein, und werden in einem späteren Verfahrensschritt auf die fertige Form weiterbearbeitet. Die Öffnungen A der Zwischenstufe Z11 werden beispielsweise durch Bohren und/oder Fräsen hergestellt. Die Öffnungen A können vor oder nach dem Heraustrennen der Zwischenstufe Z11 aus dem Rohmaterial R hergestellt werden.

Fig. 10 zeigt die plane Zwischenstufe Z11 nach der Entnahme aus dem Rohmaterial R. In dieser Darstellung ist deutlich zu erkennen, dass die Zwischenstufe Z11 plan ist, also noch nicht die gewünschte nicht-plane Form der Osteosyntheseplatte P aufweist.

Fig. 11 bis Fig. 13 zeigt verschiedene Ansichten einer nicht-planen Zwischenstufe Z12 der Osteosyntheseplatte. Diese nicht-plane Zwischenstufe Z12 wird in einem dritten Verfahrensschritt zur Herstellung der Osteosyntheseplatte P durch Umformen der planen Zwischenstufe Z11 erzeugt. Wie in den Darstellungen in Fig. 11 bis Fig. 13 deutlich zu erkennen ist, bleibt der Schichtaufbau bei der Umformung der planen Zwischenstufe Z11 zur nicht-planen Zwischenstufe Z12 erhalten.

In einem vierten Verfahrensschritt zur Herstellung der Osteosyntheseplatte P erfolgt eine Wärmebehandlung der nicht-planen Zwischenstufe Z12. Da sich die äußere Form der Zwischenstufe Z12 dabei nicht erkennbar ändert, ist die Zwischenstufe der Osteosyntheseplatte P nach dieser Wärmebehandlung nicht in den Figuren dargestellt. Durch die Wärmebehandlung werden Spannungen der nicht-planen Zwischenstufe Z12 abgebaut, welche durch die Umformung hervorgerufen worden sind. Dadurch wird ein späteres Lösen der Deckschichten S1, S2 von der Kernschicht K vermieden.

In einem zusätzlichen Verfahrensschritt nach der Wärmebehandlung werden Fasen an den Kanten hergestellt, sodass die in Fig. 1 und Fig. 2 dargestellte Osteosyntheseplatte P hergestellt wird. Zusätzlich kann eine Oberflächenbehandlung und/oder eine Oberflächenbeschichtung der Osteosyntheseplatte P vorgenommen werden, beispielsweise Schleifen, Kugelstrahlen oder Anodisieren.

Fig. 14 zeigt in der oberen Darstellung eine Draufsicht auf ein planes Rohmaterial R, und in der unteren Darstellung eine isometrische Ansicht des Rohmaterials R, wobei eine Ecke des Rohmaterials R in vergrößerter Darstellung wiedergegeben ist. Das Rohmaterial R weist bereits den mehrschichtigen Aufbau mit Kernschicht K sowie erster und zweiter Deckschicht S1, S2 auf. In einem ersten Verfahrensschritt zur Herstellung der Osteosyntheseplatte P wird ein derartiges Rohmaterial R bereitgestellt.

Fig. 15 zeigt in der oberen Darstellung eine Draufsicht auf das plane Rohmaterial R, aus dem eine plane Zwischenstufe Z21 herausgetrennt wurde. Die untere Darstellung von Fig. 15 zeigt eine entsprechende isometrische Ansicht. In einem zweiten Verfahrensschritt zur Herstellung der Osteosyntheseplatte P erfolgt ein derartiges Trennen der Zwischenstufe Z21 aus dem Rohmaterial R. Das Heraustrennen der planen Zwischenstufe Z21 kann beispielsweise durch Wasserstrahlschneiden oder Laserschneiden erfolgen. Die Zwischenstufe Z21 weist den Grundkörper G der Osteosyntheseplatte P sowie einen Spannabschnitt SA auf, welcher über zwei Verbindungsstege SV mit dem Grundkörper G verbunden ist. Die Zwischenstufe Z21 weist im Unterschied zur in Fig. 9 dargestellten Zwischenstufe Z11 keine Öffnungen im Grundkörper G auf.

Fig. 16 zeigt die plane Zwischenstufe Z21 nach der Entnahme aus dem Rohmaterial R. In dieser Darstellung ist deutlich zu erkennen, dass die Zwischenstufe Z21 plan ist, also noch nicht die gewünschte nicht-plane Form der Osteosyntheseplatte P aufweist.

Fig. 17 zeigt und Fig. 18 zeigen je eine Ansicht einer nicht-planen Zwischenstufe Z22 der Osteosyntheseplatte P. Diese nicht-plane Zwischenstufe Z22 wird in einem dritten Verfahrensschritt zur Herstellung der Osteosyntheseplatte P durch Umformen der planen Zwischenstufe Z21 erzeugt. Wie in den Darstellungen in Fig. 17 und Fig. 18 deutlich zu erkennen ist, bleibt der Schichtaufbau bei der Umformung der planen Zwischenstufe Z21 zur nicht-planen Zwischenstufe Z22 erhalten. Der Spannabschnitt SA ist in der nicht-planen Zwischenstufe Z22 weiterhin erhalten, und kann bei der Umformung zum Gegenhalten oder Greifen des Grundkörpers G verwendet werden.

In einem vierten Verfahrensschritt zur Herstellung der Osteosyntheseplatte P erfolgt eine Wärmebehandlung der nicht-planen Zwischenstufe Z22. Da sich die äußere Form der Zwischenstufe Z22 dabei nicht erkennbar ändert, ist die Zwischenstufe der Osteosyntheseplatte P nach dieser Wärmebehandlung nicht in den Figuren dargestellt. Durch die Wärmebehandlung werden Spannungen der nicht-planen Zwischenstufe Z22 abgebaut, welche durch die Umformung hervorgerufen worden sind. Dadurch wird ein späteres Lösen der Deckschichten S1, S2 von der Kernschicht K vermieden.

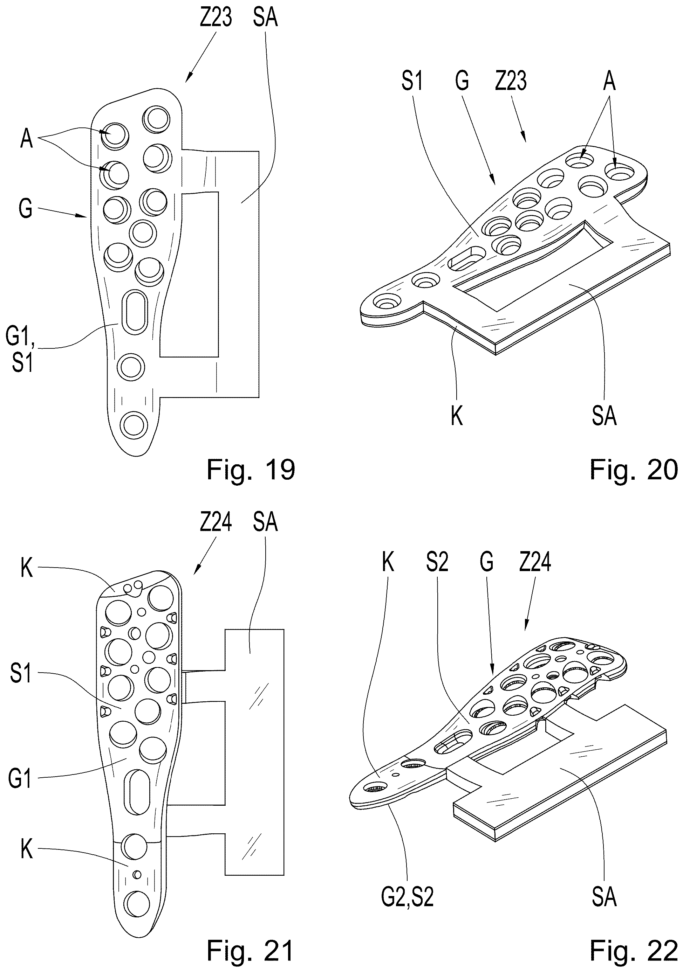

Fig. 19 und Fig. 20 zeigen je eine Ansicht einer Zwischenstufe Z23, welche die Öffnungen A im Grundkörper G aufweist. Die Öffnungen A werden in einem fünften Verfahrensschritt zur Herstellung der Osteosyntheseplatte P durch spanende Bearbeitung erzeugt. Der Spannabschnitt SA ist in der Zwischenstufe Z23 weiterhin erhalten, und kann zum Spannen des Grundkörpers G bei der spanenden Bearbeitung verwendet werden.

In einem zusätzlichen Verfahrensschritt nach der Herstellung der Öffnungen A werden Fasen an den Kanten hergestellt und der Spannabschnitt SA vom Grundkörper G getrennt, sodass die in Fig. 1 und Fig. 2 dargestellte Osteosyntheseplatte P hergestellt wird. Zusätzlich kann eine Oberflächenbehandlung und/oder eine Oberflächenbeschichtung der Osteosyntheseplatte P vorgenommen werden, beispielsweise Schleifen, Kugelstrahlen oder Anodisieren.

Soll jedoch eine Osteosyntheseplatte P gemäß dem in Fig. 6 und Fig. 7 dargestellten dritten Ausführungsbeispiel hergestellt werden, so kann vor dem zusätzlichen Verfahrensschritt eine weitere spanende Bearbeitung erfolgen, bei dem zumindest Teile der Deckschichten S1, S2 abgetragen werden. Eine bei einer solchen Bearbeitung entstehende Zwischenstufe Z24 ist in Fig. 21 und Fig. 22 dargestellt. Der Spannabschnitt SA ist in der Zwischenstufe Z24 weiterhin erhalten, und kann zum Spannen des Grundkörpers G bei der spanenden Bearbeitung verwendet werden. Anschließend werden Fasen an den Kanten hergestellt, und der Spannabschnitt SA wird vom Grundkörper G getrennt, sodass als Endprodukt die in Fig. 6 und Fig. 7 dargestellte Osteosyntheseplatte P bereitgestellt wird.

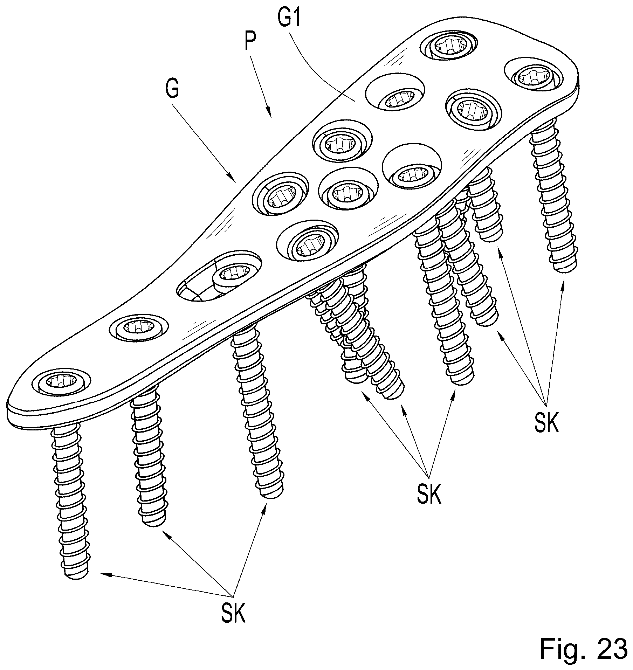

Fig. 23 zeigt eine Ansicht der Osteosyntheseplatte P gemäß dem in Fig. 1 bis Fig. 4 dargestellten zweiten Ausführungsbeispiel. In dieser Ansicht sind die Schrauben SK dargestellt, welche in den Öffnungen A im Grundkörper G der Osteosyntheseplatte P angeordnet sind. Zur Klarstellung sei festgehalten, dass nicht in jede der Öffnungen A eine Schraube SK angeordnet sein muss, um die Osteosyntheseplatte P am Knochen zu befestigen.

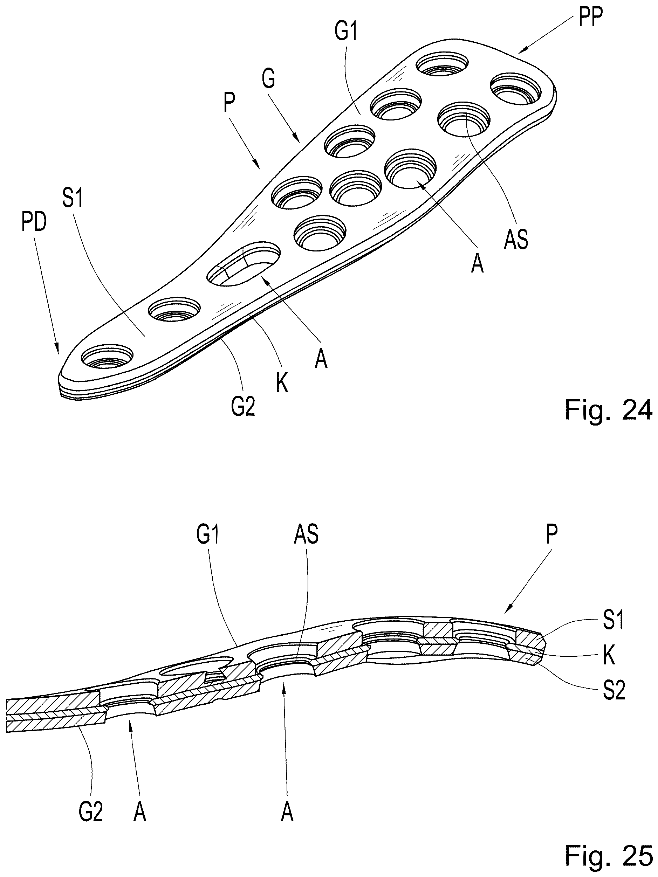

Fig. 24 zeigt eine isometrische Ansicht einer Osteosyntheseplatte P gemäß einem vierten Ausführungsbeispiel, welches im Wesentlichen dem in Fig. 1 bis 4 dargestellten ersten Ausführungsbeispiel entspricht. Allerdings ist die Kernschicht K in diesem fünften Ausführungsbeispiel dünner ausgeführt als die Deckschichten S1, S2. Um die Schultern AS der Öffnungen A bei einer derart dünnen Kernschicht K vollständig in der Kernschicht K anordnen zu können, sind die Schultern AS parallel zur Erstreckungsrichtung der Kernschicht K ausgerichtet.

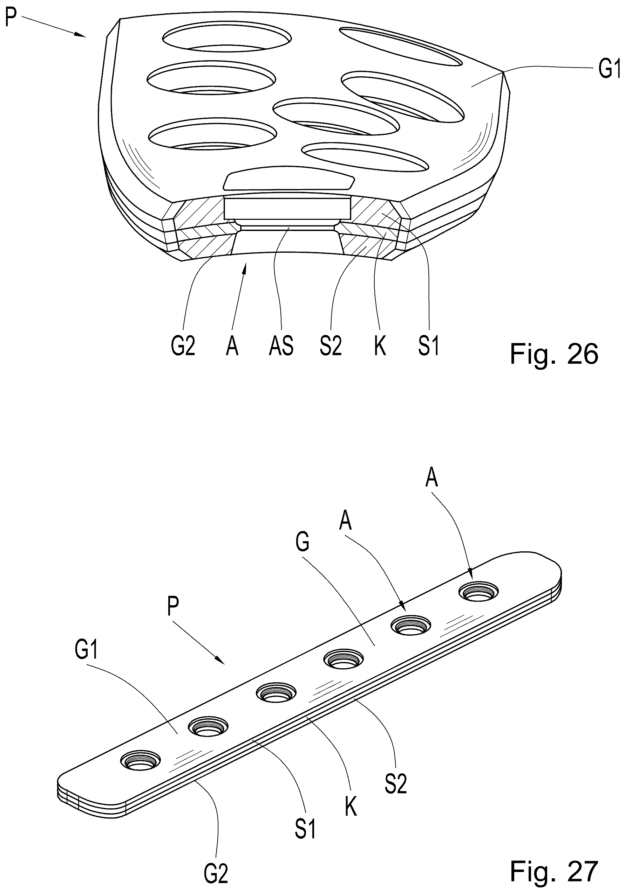

Fig. 25 und Fig. 26 zeigen je eine Schnittansicht der in Fig. 24 dargestellten Osteosyntheseplatte P. Aus diesen Ansichten gehen die im Vergleich zum in Fig. 1 bis 4 dargestellten ersten Ausführungsbeispiel geänderten Schichtdicken der Kernschicht K sowie der Deckschichten S1, S2 deutlich hervor. Auch die Ausrichtung der Schultern AS parallel zur Erstreckungsrichtung der Kernschicht K ist in Fig. 25 und Fig. 26 deutlich zu erkennen.

Fig. 27 zeigt eine isometrische Ansicht einer Osteosyntheseplatte P gemäß einem fünften Ausführungsbeispiel, welches einen völlig anderen Aufbau aufweist als die bisher beschriebenen Ausführungsbeispiele. Die Osteosyntheseplatte P gemäß dem fünften Ausführungsbeispiel weist einen länglichen Grundkörper G mit entlang seiner Erstreckungsrichtung gleichbleibendem Querschnitt auf. Der gleichbleibende Querschnitt wird nur durch Abrundungen an den Enden sowie durch die Öffnungen A unterbrochen, sodass eine derartige Osteosyntheseplatte P verhältnismäßig einfach herzustellen ist.

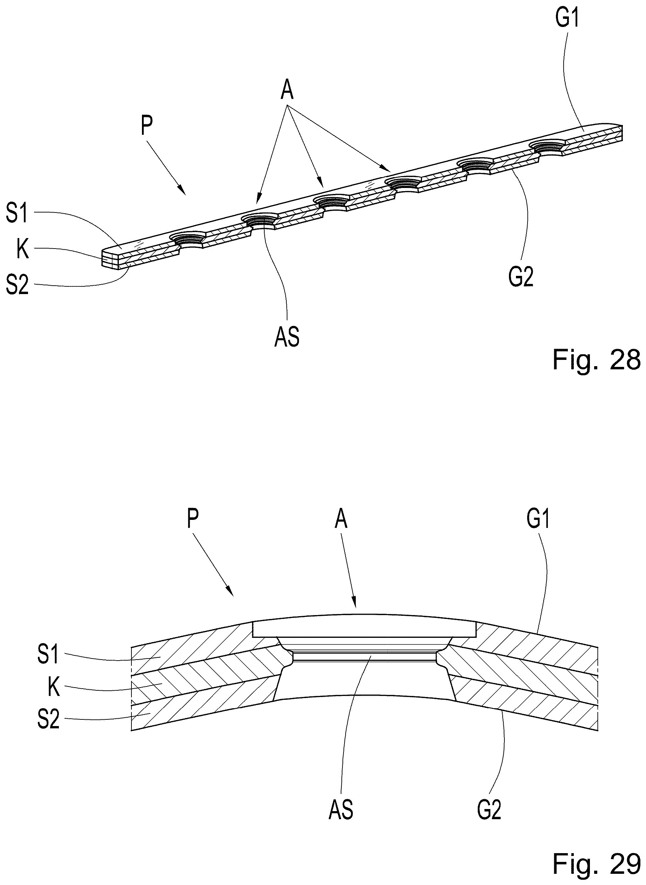

Fig. 28 zeigt eine Schnittansicht der in Fig. 27 dargestellten Osteosyntheseplatte P. Aus dieser Schnittansicht geht deutlich hervor, dass die Kernschicht K und die Deckschichten S1, S2 weitgehend die gleiche Schichtdicke aufweisen, die entlang der Längserstreckung der Osteosyntheseplatte P bis auf die Öffnungen A konstant ist. Die Schultern AS der Öffnungen A sind vollständig in der Kernschicht K angeordnet, indem die Schultern AS parallel zur Erstreckungsrichtung der Kernschicht K ausgerichtet sind.

Fig. 29 zeigt eine weitere Schnittansicht der in Fig. 27 und Fig. 28 dargestellten Osteosyntheseplatte P, wobei die Schnittebene quer zur Längserstreckung der Osteosyntheseplatte P ausgerichtet ist. Aus dieser weiteren Schnittansicht geht deutlich hervor, dass die Osteosyntheseplatte P eine nicht-plane Form aufweist. Die Osteosyntheseplatte P weist an der Unterseite G2 eine konkave Wölbung auf, um eine gute Auflagefläche auf eine runde Knochengeometrie zu gewährleisten. Um einen gleichbleibenden Querschnitt zu erhalten, weist die Oberseite G1 eine entsprechende konvexe Wölbung auf. Die Geometrie der Öffnung A inklusive der Geometrie der Schulter AS ist durch spanabhebende Bearbeitung erzeugt, sodass die Schichtdicke der Kernschicht K und der Deckschichten S1, S2 im Bereich der Öffnungen A nicht konstant ist. Die nicht-plane Form der Osteosyntheseplatte P wird durch Umformen eines Rohmaterials erzeugt, welcher bereits den Schichtaufbau mit Kernschicht K und Deckschichten S1, S2 umfasst. Die Geometrie der Schulter AS wird erst nach dem Umformprozess erzeugt.

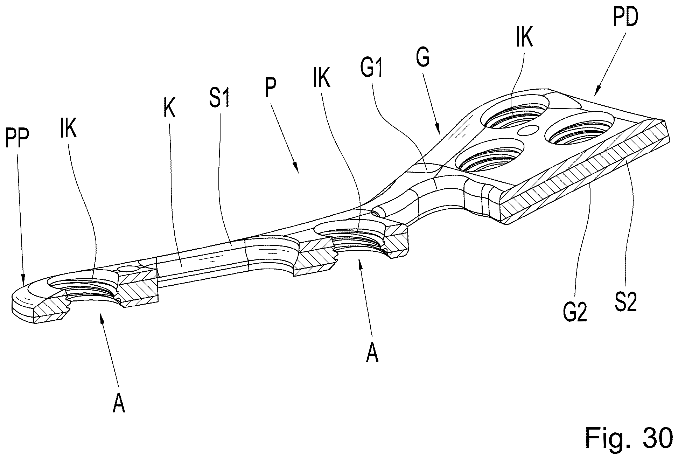

Fig. 30 zeigt eine Schnittansicht eines sechsten Ausführungsbeispiels einer Osteosyntheseplatte P, welches zur Stabilisierung einer distalen Radiusfraktur ausgebildet ist. Das distale Ende PD der Osteosyntheseplatte P ist im Bereich des Handgelenks aufzulegen, während das proximale Ende PP im Bereich der Diaphyse des Radius angeordnet ist. Die Osteosyntheseplatte P weist eine Kernschicht K und Deckschichten S1, S2 mit jeweils konstanter Schichtdicke auf. In den Öffnungen A der Osteosyntheseplatte P, welche zur Aufnahme von in Fig. 30 nicht dargestellten Schrauben ausgebildet sind, ist jeweils ein Innengewinde IK ausgebildet.

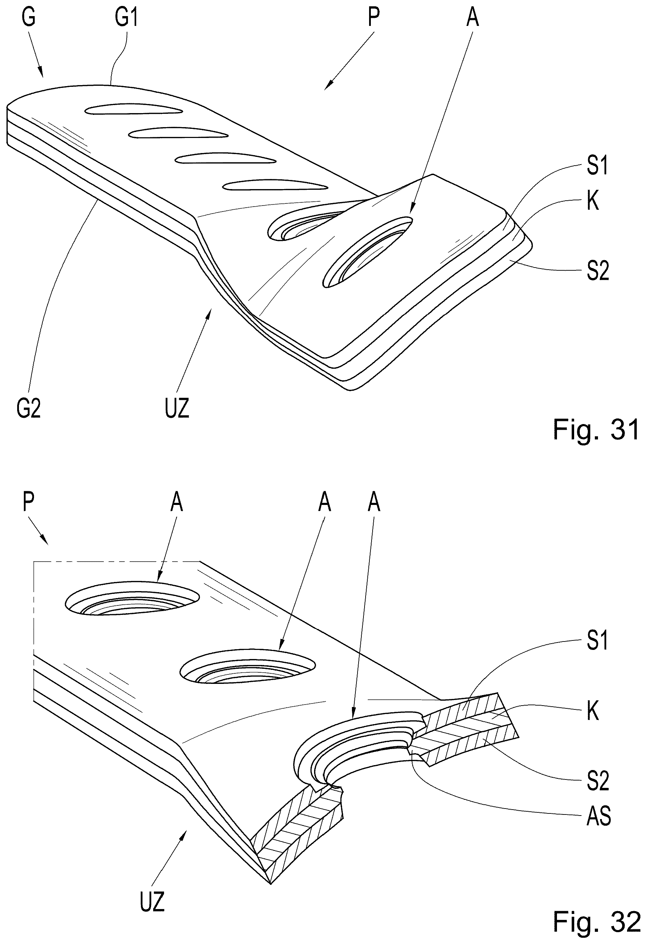

Fig. 31 und Fig. 32 zeigen je eine Ansicht einer Osteosyntheseplatte P gemäß einem siebenten Ausführungsbeispiel, welches im Gegensatz zum in Fig. 30 dargestellten Ausführungsbeispiel in den Öffnungen A keine Innengewinde aufweist. Stattdessen weisen die Öffnungen A eine Schulter AS zur Aufnahme einer in Fig. 31 und Fig. 32 nicht dargestellten Schraube auf. Die Osteosyntheseplatte P gemäß dem siebenten Ausführungsbeispiel weist eine Umformzone UZ auf. In dieser Umformzone UZ wird die Osteosyntheseplatte P plastisch verformt, um eine anatomisch vorteilhafte Geometrie auszubilden. Die Geometrie der Öffnungen A inklusive der Schulter AS ist dabei vor dem Umformprozess eingebracht worden, sodass die während des Umformprozesses verformte Schulter AS eine nicht-rotationssymmetrische Form aufweist. Dies ist in übertriebener Form in der Schnittdarstellung gemäß Fig. 32 gut zu erkennen.

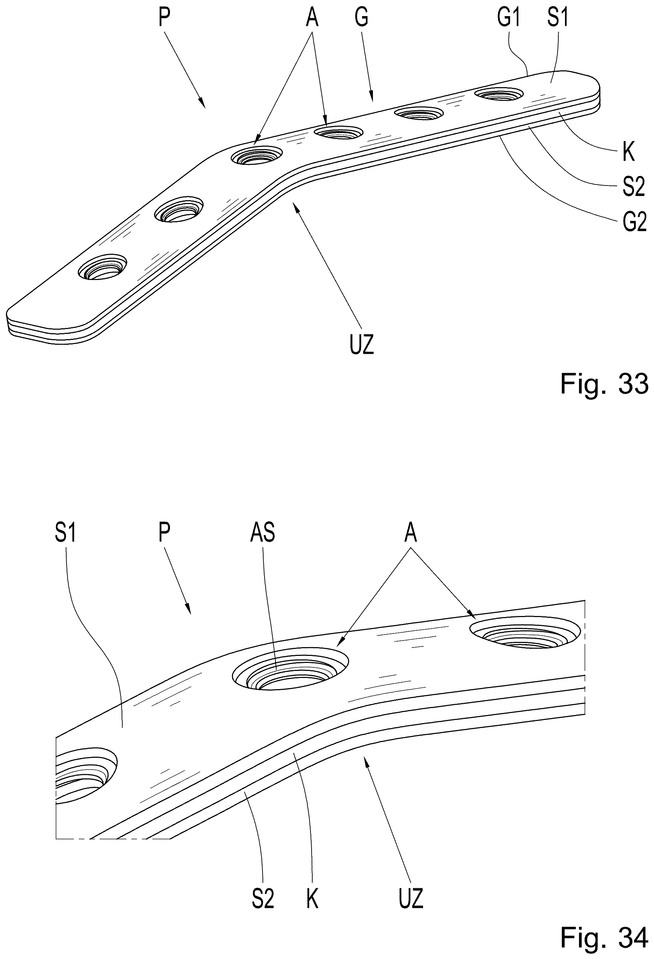

Fig. 33 und Fig. 34 zeigen je eine Ansicht einer Osteosyntheseplatte P gemäß einem achten Ausführungsbeispiel, wobei in ähnlicher Weise wie im in Fig. 31 und Fig. 32 dargestellten siebenten Ausführungsbeispiel eine Umformzone UZ vorliegt. Die Osteosyntheseplatte P gemäß dem achten Ausführungsbeispiel ist dabei an einer Stelle entlang ihrer Längserstreckung in nur einer Ebene verformt. Eine zusätzliche Torsion entlang der Längserstreckung ist hier nicht vorgenommen. Eine der Öffnungen A befindet sich in der Umformzone UZ. Die Geometrie der Öffnungen A ist vor dem Umformprozess eingebracht worden, sodass die während des Umformprozesses verformte Schulter AS eine nicht-rotationssymmetrische Form aufweist.

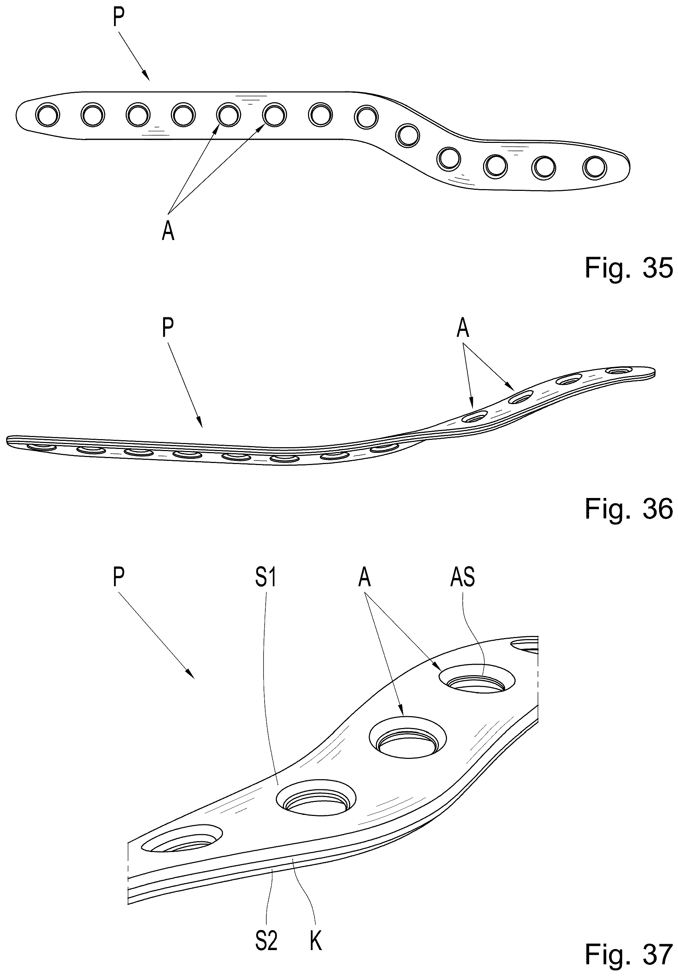

Fig. 35 bis Fig. 37 zeigen je eine Ansicht einer Osteosyntheseplatte P gemäß einem neunten Ausführungsbeispiel, welche einen dünneren Querschnitt als die Osteosyntheseplatte P gemäß dem siebenten und achten Ausführungsbeispiel aufweist. Dadurch kann die Osteosyntheseplatte P gemäß dem neunten Ausführungsbeispiel entlang ihrer gesamten Erstreckung dreidimensional verformt werden, sodass eine optimale Anpassung an eine Anatomie gegeben ist. Die Öffnungen A inklusive der Schulter AS werden bevorzugt vor dem Umformprozess in die Osteosyntheseplatte P eingebracht. Alternativ dazu können die Öffnungen, bzw. die Geometrie der Schultern AS erst nach dem Umformprozess hergestellt werden.

### Bezugszeichenliste

- P: Osteosyntheseplatte
- PP: proximales Ende
- PD: distales Ende
- G: Grundkörper
- G1: Oberseite
- G2: Unterseite
- A: Öffnung
- AS: Schulter
- IK: Innengewinde
- K: Kernschicht
- S1: Erste Deckschicht
- S2: Zweite Deckschicht
- NA: Nahtanker
- SK: Schraube
- R: Rohmaterial
- Z11: Zwischenstufe
- Z12: Zwischenstufe
- Z21: Zwischenstufe
- Z22: Zwischenstufe
- Z23: Zwischenstufe
- Z24: Zwischenstufe
- SA: Spannabschnitt
- SV: Verbindungsstege
- UZ: Umformzone

## Patentansprüche

1. Osteosyntheseplatte (P) zur Fixierung von Knochenfragmenten, wobei die Osteosyntheseplatte (P) einen Grundkörper (G) mit einer Oberseite (G1) und mit einer der Oberseite (G1) gegenüberliegenden Unterseite (G2) aufweist, wobei die Unterseite (G2) zur Auflage der Osteosyntheseplatte (P) auf den Knochenfragmenten vorgesehen ist, wobei die Osteosyntheseplatte (P) mehrere zwischen der Oberseite (G1) und der Unterseite (G2) ausgebildete Öffnungen (A) zur Aufnahme von je einer Schraube (SK) aufweist,
**dadurch gekennzeichnet, dass** der Grundkörper (G) einen mehrschichtigen Aufbau aufweist, umfassend eine Kernschicht (K), eine erste Deckschicht (S1), welche die Kernschicht (K) auf einer ersten Seite zumindest teilweise bedeckt, und eine zweite Deckschicht (S2), welche die Kernschicht (K) auf einer der ersten Seite gegenüberliegenden zweiten Seite zumindest teilweise bedeckt, wobei die Kernschicht (K) aus einem ersten Werkstoff gefertigt ist, wobei die erste und zweite Deckschicht (S1, S2) aus einem zweiten Werkstoff gefertigt sind, wobei sich der erste Werkstoff vom zweiten Werkstoff unterscheidet, und wobei der erste Werkstoff eine höhere Duktilität aufweist als der zweite Werkstoff.

2. Osteosyntheseplatte (P) nach Anspruch 1, **dadurch gekennzeichnet, dass** die Kernschicht (K) aus Reintitan gebildet ist, vorzugsweise aus Titan Grade 1, oder aus Titan Grade 2, oder aus Titan Grade 3 oder aus Titan Grade 4, und wobei die erste und zweite Deckschicht (S1, S2) aus einer Titanlegierung, vorzugsweise aus Ti6Al4V oder aus TiAl6Nb7, oder aus Stahl gebildet ist, vorzugsweise aus X2CrNiMo18-15-3, oder aus X2CrMnMo 18-13-3, oder aus X4CrNiMnMo 21-9-4, oder aus CoCr28Mo6 mit einem Kohlenstoffgehalt größer 0,2 %.

3. Osteosyntheseplatte (P) nach Anspruch 1, **dadurch gekennzeichnet, dass** die Kernschicht (K) aus einer ersten Stahlsorte, vorzugsweise aus CoCr28Mo6 mit einem Kohlenstoffgehalt kleiner 0,055 %, oder aus X2CrNiMo18-15-3, oder aus X4CrNiMnMo 21-9-4 oder aus X2CrMnMo 18-13-3 gebildet ist, wobei die erste und zweite Deckschicht (S1, S2)
- aus einer zweiten Stahlsorte, vorzugsweise aus CoCr28Mo6 mit einem Kohlenstoffgehalt größer 0,2 %, oder aus X2CrNiMo18-15-3, oder aus X4CrNiMnMo 21-9-4 oder aus X2CrMnMo 18-13-3, oder
- aus einer Titanlegierung gebildet ist, vorzugsweise TiAl6Nb7 oder Ti6Al4V.

4. Osteosyntheseplatte (P) nach einem der Ansprüche 1 bis 3, **dadurch gekennzeichnet, dass** in zumindest einer der Öffnungen (A) eine Schulter (AS) zur Abstützung der Schraube (SK) ausgebildet ist, wobei die Schulter (AS) in der Kernschicht (K) angeordnet ist.

5. Osteosyntheseplatte (P) nach einem der Ansprüche 1 bis 4, **dadurch gekennzeichnet, dass** in zumindest einer der Öffnungen (A) ein Innengewinde (IK) ausgebildet ist, welches entweder ausschließlich oder zu einem überwiegenden Anteil in der Kernschicht (K) ausgebildet und dazu eingerichtet ist, mit einem Außengewinde der Schraube (SK) zusammenzuwirken.

6. Osteosyntheseplatte (P) nach einem der Ansprüche 1 bis 5, **dadurch gekennzeichnet, dass** die Kernschicht (K), die erste Deckschicht (S1) und/oder die zweite Deckschicht (S2) eine konstante Schichtdicke aufweisen.

7. Osteosyntheseplatte (P) nach einem der Ansprüche 1 bis 6, **dadurch gekennzeichnet, dass** die erste Deckschicht (S1) die gleiche Schichtdicke wie die zweite Deckschicht (S2) aufweist.

8. Osteosyntheseplatte (P) nach einem der Ansprüche 1 bis 5, **dadurch gekennzeichnet, dass** die Kernschicht (K) eine konstante Schichtdicke aufweist, wobei die erste Deckschicht (S2) und/oder die zweite Deckschicht (S2) eine nicht-konstante Schichtdicke aufweisen.

9. Osteosyntheseplatte (P) nach einem der Ansprüche 1 bis 8, **dadurch gekennzeichnet, dass** die erste Deckschicht (S1) ausschließlich an der Oberseite (G1), und die zweite Deckschicht (S2) ausschließlich an der Unterseite (G2) ausgebildet ist.

10. Osteosyntheseplatte (P) nach Anspruch 9, **dadurch gekennzeichnet, dass** die erste Deckschicht (S1) und/oder die zweite Deckschicht (S2) die Kernschicht (K) an der Oberseite (G1) bzw. an der Unterseite (G2) vollflächig bedeckt.

11. Osteosyntheseplatte (P) nach Anspruch 9 oder Anspruch 10, **dadurch gekennzeichnet, dass** die Schichtdicke der ersten und/oder der zweiten Deckschicht (S1, S2) zwischen 0,2 und 2 mm beträgt.

12. Osteosyntheseplatte (P) nach Anspruch 9, **dadurch gekennzeichnet, dass** die erste Deckschicht (S1) und/oder die zweite Deckschicht (S2) die Kernschicht (S1) nur abschnittsweise bedeckt, sodass die Kernschicht (K) an der Oberseite (G1) und/oder an der Unterseite (G2) zumindest teilweise freiliegt.

13. Osteosyntheseplatte (P) nach einem der Ansprüche 9 bis 12, **dadurch gekennzeichnet, dass** die Kernschicht (K) eine zumindest 1,5 mal, vorzugsweise dreimal so hohe Schichtdicke aufweist wie die erste und zweite Deckschicht (S1, S2) an ihrer dicksten Stelle.

14. Osteosyntheseplatte (P) nach Anspruch 13 unter Rückbezug auf Anspruch 4, **dadurch gekennzeichnet, dass** die in der Kernschicht (K) angeordnete Schulter (AS) zur Abstützung der Schraube (SK) schräg zu einer Erstreckungsrichtung der Kernschicht (K) ausgerichtet ist.

15. Osteosyntheseplatte (P) nach einem der Ansprüche 9 bis 12, **dadurch gekennzeichnet, dass** die Kernschicht (K) entweder eine geringere oder eine maximal 1,5 mal so hohe Schichtdicke aufweist wie die erste und zweite Deckschicht (S1, S2) an ihrer dicksten Stelle.

16. Osteosyntheseplatte (P) nach Anspruch 15 unter Rückbezug auf Anspruch 4, **dadurch gekennzeichnet, dass** die in der Kernschicht (K) angeordnete Schulter (AS) zur Abstützung der Schraube (SK) parallel zu einer Erstreckungsrichtung der Kernschicht (K) ausgerichtet ist.

17. Osteosyntheseplatte (P) nach einem der Ansprüche 1 bis 8, **dadurch gekennzeichnet, dass** sich die erste Deckschicht (S1) und die zweite Deckschicht (S2) entlang von gegenüberliegenden Seitenwänden der Osteosyntheseplatte (P) erstrecken, sodass sich die Kernschicht (K), die erste Deckschicht (S1) und die zweite Deckschicht (S2) von der Oberseite (G1) bis zur Unterseite (G2) erstrecken, wobei die Schichtdicke der ersten und/oder der zweiten Deckschicht (S1, S2) zwischen 0,5 und 5 mm beträgt.

18. Osteosyntheseplatte (P) nach einem der Ansprüche 1 bis 17, **dadurch gekennzeichnet, dass** die Osteosyntheseplatte (P) eine längliche nicht-plane Form aufweist.

19. Verfahren zur Herstellung einer Osteosyntheseplatte (P) gemäß einem der Ansprüche 1 bis 18, **gekennzeichnet durch** folgende Schritte:
- erster Schritt: Bereitstellen eines planen Rohmaterials (R), welches die Kernschicht (K) sowie die erste und zweite Deckschicht (S1, S2) aufweist,
- zweiter Schritt: Trennen einer Zwischenstufe (Z11, Z21) der Osteosyntheseplatte (P) aus dem Rohmaterial (R),
- dritter Schritt: Umformen, um eine gewünschte nicht-plane Form der Osteosyntheseplatte (P) zu erzielen,
- vierter Schritt: Wärmebehandeln der nicht-planen Form der Osteosyntheseplatte (P), und optionaler
- zusätzlicher Schritt: Oberflächenbearbeitung und/oder Oberflächenbeschichtung der im vierten Schritt wärmebehandelten Osteosyntheseplatte (P).

20. Verfahren zur Herstellung einer Osteosyntheseplatte (P) nach Anspruch 19, **dadurch gekennzeichnet, dass** der zweite Schritt vor dem dritten Schritt durchgeführt wird, sodass die aus dem Rohmaterial (R) getrennte Zwischenstufe (Z11, Z21) eine plane Form aufweist, oder dass der zweite Schritt nach dem dritten Schritt durchgeführt wird, sodass die aus dem Rohmaterial (R) getrennte Zwischenstufe die gewünschte nicht-plane Form aufweist.

21. Verfahren zur Herstellung einer Osteosyntheseplatte (P) nach Anspruch 19 oder Anspruch 20, **dadurch gekennzeichnet, dass** in einem fünften Schritt eine spanende Bearbeitung zur Herstellung einer Geometrie der Öffnungen (A) der Osteosyntheseplatte (P) durchgeführt wird, wobei der fünfte Schritt unmittelbar nach dem zweiten, dritten oder vierten Schritt durchgeführt wird.
